# EUROPÄISCHE PATENTSCHRIFT

(11) **EP 2 299 264 B1**
(45) Veröffentlichungstag und Bekanntmachung des Hinweises auf die Patenterteilung: **20.06.2018**
(21) Anmeldenummer: 10009828.4
(22) Anmeldetag: 17.09.2010
(51) Int. Cl.: G01N 33/00, G01N 29/024, G01N 29/22

(54) **Vorrichtung zur Bestimmung eines Gasanteiles in einem Gasgemisch**
Device for measuring a gas content in a gas mixture
Dispositif destiné à la détermination d'une teneur en gaz dans un mélange de gaz

(30) Priorität: 18.09.2009 DE 102009041831; 05.02.2010 DE 102010006901
(43) Veröffentlichungstag der Anmeldung: 23.03.2011
(73) Patentinhaber: Firma DILO Armaturen und Anlagen GmbH, 87727 Babenhausen (DE)
(72) Erfinder: Marz, Philipp, D-87755 Kirchhaslach (DE)
(74) Vertreter: Pfister, Stefan Helmut Ulrich

(56) Entgegenhaltungen:
- WO-A1-2006/133738
- US-A- 5 392 635
- US-B1- 6 279 378
- US-B2- 7 184 895
- HAQUE E ET AL: "Application of acoustic sensing and signal processing for PD detection in GIS", INFORMATION, COMMUNICATIONS AND SIGNAL PROCESSING, 1997. ICICS., PROCE EDINGS OF 1997 INTERNATIONAL CONFERENCE ON SINGAPORE 9-12 SEPT. 1997, NEW YORK, NY, USA,IEEE, US, 9. September 1997 (1997-09-09), Seiten 745-749, XP010263954, DOI: 10.1109/ICICS.1997.652077 ISBN: 978-0-7803-3676-6

## Beschreibung

Die Erfindung betrifft eine Vorrichtung zur Bestimmung des Anteiles eines Gases in einem Gasgemisch, insbesondere von SF6-beziehungsweise CF4-Gas in einem Gasgemisch.

SF6- beziehungsweise CF4-Gas sind als Füllgas von elektrischen Schalteinrichtungen bekannt. Diese Gase zeichnen sich durch ihre hohe Durchschlagsfestigkeit aus, das heißt, Funkenüberschläge zwischen zwei Elektroden können durch die Verwendung der vorgenannten Gase stark reduziert werden, was zu einem entsprechenden Schutz der Elektroden führt.

Allerdings beeinträchtigt der Anteil von Fremdgasen wie zum Beispiel Luft oder Stickstoff oder anderen Gasen diese Durchschlagsfestigkeit.

Aus dem Stand der Technik sind unterschiedliche Vorrichtungen und Verfahren zur Bestimmung der Konzentration von Komponenten eines Gasgemisches bekannt. Bekannt ist es auch beispielsweise mittels einer entsprechenden Vorrichtung oder eines Verfahrens die charakteristischen Bestandteile der Atemluft von Lebewesen zu bestimmen. Des Weiteren ist es aus dem Stand der Technik bekannt, den Gasanteil in einer Flüssigkeit zu messen. Ein anderes Verfahren bestimmt den Gesamtanteil eines Gases gegenüber einem sonstigen inerten Bestandteil eines Gasstromes.

Die US-Patentschrift US 6,279,378 B1 beschreibt ein auf Ultraschall basierendes Analysegerät sowie ein Analyseverfahren zum Auffinden von Spurengasen. Dabei sollen Spuren von Helium in Luft aufgefunden werden. Das beschriebene Gerät misst dafür die Laufzeit von Schallwelle zwischen einer Schallquelle und einem Detektor und vergleicht die Laufzeit mit einem Referenzwert der Laufzeit in reiner Luft.

In US 5,392,635 A wird eine akustische Analyse von Gasgemischen offenbart. Dabei kommt die Pitch-and-Catch Methode zum Einsatz um ein günstiges Verhältnis zwischen Signal und Rauschen zu erhalten. Das Messprinzip ist auch hier eine Messung der Laufzeit von Schallwellen.

Die Messung der Laufzeiten von Schallwellen zur Gasanalyse weist bei Füllgasen für Schalteinrichtungen allerdings Nachteile auf.

Es ist daher Aufgabe der vorliegenden Erfindung eine Vorrichtung zur Bestimmung des Anteiles, insbesondere von SF6- und/oder CF4-Gasen in einem Gasgemisch zur Verfügung zu stellen.

Zur Lösung dieser Aufgabe geht die Erfindung aus von einer Vorrichtung zur Bestimmung des Anteiles von SF6- beziehungsweise CF4-Gas (dabei ist es mit der Erfindung möglich, sowohl den Anteil von SF6 als auch den Anteil von CF4-Gas in einem binären Gasgemisch zu bestimmen), wobei sich das Gasgemisch in einer Messzelle befindet und an der Messzelle eine Schallquelle und mindestens ein Schallempfänger angeordnet sind. Der mindestens eine Schallempfänger nimmt die von der Schallquelle abgestrahlte Schallwelle auf und leitet dieses Empfangssignal einer Auswerteinheit zur Bestimmung der Schallgeschwindigkeit und daraus resultierend zur Bestimmung des Anteils von SF6- beziehungsweise CF4-Gas zu. Erfindungsgemäß sind, im Unterschied zum Stand der Technik, mindestens zwei, untereinander und auch von der Schallquelle entfernte, phasenempfindliche Schallempfänger an der Messzelle vorgesehen sind, die Schallwellen phasenaufgelöst empfangen und das jeweilige Empfangssignal der Auswerteeinheit zuleiten und die Auswerteeinheit aus dem Phasenunterschied der jeweiligen Empfangssignale die Schallgeschwindigkeit und hieraus den Anteil des jeweiligen Gases bestimmt.

Entgegen allen Lösungen, die bisher im Stand der Technik zur Messung von bestimmten Gasanteilen in Gasgemischen und/oder anderen Gemischen zu bestimmen, geht die Erfindung hier einen völlig neuen Weg, indem nämlich eine Messzelle vorgesehen ist, innerhalb welcher dann die Messung stattfindet. Die Messzelle ist dabei unabhängig von dem Gasstrom des Mediums insgesamt geschaltet und/oder angeordnet. Sie kann sich beispielsweise außerhalb des Gasstromes oder des Gasbehälters befinden, dessen Inhalt zu messen ist. Sie kann sich allerdings genauso in der Zuleitung zu einer entsprechenden Anlage befinden, die mit SF6 oder mit CF4-Gas beschickt werden soll. Dabei ist der Grundgedanke der, dass man eine bestimmte Gasmenge in die Messzelle hineinströmen lässt, dann misst, und dann das gemessene Gas wieder abströmen lässt, um beispielsweise eine erneute Prüfung vorzunehmen oder aber um ein Spülgas einzuspülen oder dergleichen. Selbstverständlich ist es nach der Erfindung auch möglich, einen wenn auch sehr geringen Gasdurchflussstrom innerhalb der Messzelle zu messen. Dazu wird allerdings die Strömungsgeschwindigkeit reduziert, was weiter hinten dann noch ausführlich beschrieben werden wird.

Die Erfindung schlägt eine Messzelle mit den Merkmalen des Anspruchs 1 vor, innerhalb derer die Messung vorgenommen wird. Dies schließt nicht aus, dass auch dort eine geringe Strömung vorhanden ist, die insbesondere mit gezielten Maßnahmen reduziert werden kann. Allerdings ist eben ein von dem normalen Medienstrom getrenntes Vorgehen ausschlaggebend, um hier höhere Genauigkeiten zu erhalten. Auch ist es dadurch möglich, ggf. eine portable Vorrichtung zu erhalten, die an unterschiedlichen Messorten einsetzbar ist.

Durch die Verwendung der Schallgeschwindigkeit zur Bestimmung des Anteiles von SF6- beziehungsweise SF4-Gas in einem Gas wird eine auf der einen Seite ausreichend empfindliche und auf der anderen Seite auch gut messbare Kenngröße verwendet. Der erfindungsgemäße Vorschlag ist daher grundsätzlich geeignet eine Vorrichtung mit entsprechender Genauigkeit, was die Anteilsbestimmung angeht, zur Verfügung zu stellen.

Die Auswerteinheit ist als Mikrokontroller realisert und bestimmt im ersten Schritt die Schallgeschwindigkeit der Schallwelle in dem Gasgemisch. Des Weiteren umfasst die Auswerteinheit einen Speicher, in dem für die gängigen Gasgemischanteile Werte in einer Tabelle hinterlegt sind und/oder aus Formeln errechnet werden können, um aus der ermittelten Schallgeschwindigkeit auf die Gaszusammensetzung schließen zu können. In einer erfindungsgemäßen Variante liegen diese Wertetabellen auch für unterschiedliche Messdrücke und Temperaturen vor und/ oder können aus Formeln errechnet werden.

Eingangsseitig vor der Messzelle im Gaszulauf ist in einer erfindungsgemäßen Variante ein Gasanalysator vorgesehen, durch welchen zumindest qualitativ ermittelt wird, mit welchem Gasbestandteil in dem Gasgemisch zu rechnen ist, um somit den Einsatzbereich der erfindungsgemäßen Vorrichtung erheblich zu erweitern.

Erfindungsgemäß umfasst der Begriff "Gasgemisch" nicht zwingend mindestens zwei Gase, der Begriff "Gasgemisch" umfasst auch die Fälle, in welchen eine 100%ige Konzentration zum Beispiel an SF6, CF4 oder einem anderen Gas besteht.

In einer bevorzugten Variante der Erfindung ist vorgesehen, dass an der Messzelle ein Gaszulauf und ein Gasabfluss vorgesehen ist. Durch eine solche Ausgestaltung ist es grundsätzlich auch möglich, die Messung bei einem strömenden Gas, aber im Unterschied zum Stand der Technik in einer Messzelle durchzuführen. Die Strömungsgeschwindigkeit des Gases ist dabei ausreichend gering, um die Messung nicht zu behindern. Es ist auch möglich, am Gaszubeziehungsweise Gasablauf je ein Absperrventil vorzusehen und eine stationäre Messung durchzuführen, beispielsweise indem die Messzelle befüllt, nach dem Füllen die Messzelle abgesperrt und dann gemessen wird.

Vorzugsweise ist im Gaszulauf eine Einlassblende vorgesehen, durch die das Druckniveau in der Messzelle eingestellt wird. Hierbei wird insbesondere das Druckniveau auf nahzu atmosphärischen Druck eingestellt. Es ist gefunden worden, dass es günstig ist, dass während der Messung in der Messzelle ein Messdruck zwischen 1 bar und 1,7 bar, bevorzugt ca. 1,2 bar bis 1,7 bar besteht. Je nach Viskosität des Gasgemisches ergeben sich unterschiedliche Durchflussraten. Diese können je nach Vordruck schwanken. Die niedrigste Durchflussrate bei kleinstem Betriebsdruck vor der Blende wir dabei günstigerweise so eingestellt, dass in der Messzelle, beispielsweise nach ca. 3 bis 4 Minuten, ein vollkommener Gasaustausch bei dem am zähestens der verwendeten Gase (100 Vol-% SF6-Gas) stattfindet. Die im Gaszulauf verwendbare Einlassblende ist günstigerweise aus einem dünnen rostfreien Stahlblech gefertigt. Die Blende weist wenigstens eine Bohrung für den Durchtritt des Gases auf. Die Bohrung weist bevorzugt einen Durchmesser im µm-Bereich auf. Als besonders vorteilhaft wird angesehen, wenn die Blende eine bis 8, bevorzugt 4 bis 6 Bohrungen, aufweist. Dies bietet den Vorteil, dass fertigungstechnische Streuungen und damit stark unterschiedliche Durchflussraten ausgeschlossen werden können. Die Bohrungen in der Blende werden insbesondere mittels Laser in die Blende eingebracht. Die Anzahl der letztendlich gefertigten Bohrungen in der Blende ist von der Laserfokussierung und daher von dem mit dem jeweils verwendeten Laser erzielbaren Durchmesser abhängig. Mehrere Bohrungen mit geringem Durchmesser bewirken dabei die gleichen Durchflussraten wie wenige Bohrungen mit verhältnismäßig größerem Durchmesser.

Zu beachten ist, dass durch die Einlassblende generell eine entsprechende Abkühlung des Messgases erfolgen kann, insbesondere dann, wenn das Druckniveau in der Zuleitung entsprechend hoch ist. Diese Abkühlung ist in der Regel jedoch relativ klein, da die Durchflussrate sehr gering ist. In einer bevorzugten Ausführungsform ist die Messzelle aus Metall gefertigt. Hierbei ist von Vorteil, dass die Abkühlung auch bei ständigem Gasfluss unwesentlich ist, da das Metall eine entsprechende Wärmemenge aus der Umgebung aufzunehmen vermag beziehungsweise selbst eine hohe Wärmekapazität besitzt. Durch den ständigen Kontakt mit den Wänden der Messzelle erreicht das Gas praktisch das Temperaturniveau der Messzelle. Die Temperatur der Messzelle wird mit einem Temperatursensor gemessen und in der Auswerteeinheit berücksichtigt. Die Temperatur des Gases wird geschickterweise durch einen der Messzelle zugeordneten zusätzlichen Temperatursensor ermittelt. Bestehende Temperaturunterschiede zwischen Messzelle und Gastemperatur können durch geeignete Kalibrierung eliminiert werden. In einer bevorzugten Ausführungsform der Erfindung liegt ein der Messzelle zugeordneter Drucksensor vor, der den Druck des Gases erfasst und an die Auswerteeinheit weitergibt. Somit kann beispielsweise überprüft werden, dass in der Messzelle tatsächlich eine Gasmischung vorliegt und nicht die Bestandteile in ihrer flüssigen Phase.

Geschickterweise sind in dem Gaszulauf auch noch gegebenenfalls weitere Druckminderer angeordnet, um eine Abschwächung des Eingangs-Betriebsdruckes auf den erforderlichen maximalen Messdruck beziehungsweise Betriebsdruck der Vorrichtung zu erreichen.

So wird vorgesehen, dass ein Betriebsdruck in der Zuleitung zum Gaszulauf beziehungsweise im Gaszulauf, insbesondere vor der Einlassblende von bis zu 20 bar besteht, insbesondere von bis zu 15 bar. Gute Ergebnisse wurden dabei insbesondere für einen Druckbereich von ca. 0,2 bis zu 10 bar (+/- 15%) als Betriebsdruck erreicht.

Um einen Verlust des Gasgemisches, das SF6 beziehungsweise CF4 beinhaltet, zu vermeiden, wird in einer Variante eine Gasführung am Gasablauf vorgesehen.

Vorteilhafterweise wird in einer Variante eine Messung bei strömenden Gas durch die Messzelle durchgeführt. Die Fließgeschwindigkeit des Gases beziehungsweise Gasgemisches durch die Messzelle ist dabei deutlich geringer als die Schallgeschwindigkeit des Gases, sodass dadurch die Messung der Schallgeschwindigkeit nicht beeinflusst wird. Die Fließgeschwindigkeit beträgt dabei zum Beispiel maximal 0,1% der Schallgeschwindigkeit. So wird eine Durchsatzmasse durch die Messzelle von bis zu weniger als 2,4 g/Min., bevorzugt weniger als 1,8 g/Min., insbesondere weniger als 1,5 g/Min. bei 100 Vol-% SF6-Gas durch die Messzelle vorgeschlagen. Sehr gute Ergebnisse wurden dabei mit einer Durchsatzmasse von ca. 1,2 g/Min (+/- 15%) bei 100Vol-% SF6-Gas und einem Betriebsdruck von 10 bar vor der Einlassblende erreicht. Nach der Einlassblende bestand dabei ein Messdruck von ca. 1,2 bar.

Bevorzugterweise wird eine sich länglich erstreckend ausgebildete Messzelle vorgesehen. Eine länglich ausgebildete Messzelle erlaubt eine verhältnismäßig lange Messstrecke und damit eine hohe Genauigkeit bei der Bestimmung der Schallgeschwindigkeit. Üblicherweise ist die Messzelle zum Beispiel rohr- oder kapillarartig ausgebildet und besitzt einen Querschnitt. Die effektive Länge der Messzelle ist dabei mindestens 5 mal, bevorzugt mindestens 10 mal, insbesondere mindestens 20 mal größer als die den Querschnitt beschreibende Länge (zum Beispiel Durchmesser). Erfindungsgemäß wird vorgeschlagen, dass mindestens zwei, räumlich voneinander, aber auch räumlich von der Schallquelle beabstandete Schallempfänger vorgesehen sind. Somit werden als Geräte getrennte, als Schallquelle oder als Schallempfänger dienende Geräte eingesetzt. Zusätzlich kann ein Gerät sowohl als Schallquelle sowie auch als Schallempfänger dienen. Dabei wird vorgeschlagen, dass die Schallquelle insbesondere in Abstrahlpausen auch als Schallempfänger dient. Die Erfindung macht sich dabei den Vorteil zu Nutze, dass die Emission aber auch das Empfangen von Schall nach gleichartigen, jeweils umgekehrten Prinzipien erfolgt und daher eine Schallquelle grundsätzlich auch als Schallempfänger (und umgekehrt) fungieren kann.

Die beiden vorgenannten unterschiedlichen Konzepte (Schallquelle gerätetechnisch getrennt von Schallempfänger beziehungsweise ein Gerät welches als Schallquelle beziehungsweise als Schallempfänger dient) führen gemäß einer Variante auch zu zwei verschiedenen Betriebsmodi der Schallquelle. So wird erfindungsgemäß vorgeschlagen, dass eine von der Schallquelle kontinuierlich abgestrahlte Schallwelle eingesetzt wird. Die Länge der kontinuierlich abgestrahlten Schallwelle ist dabei mindestens so lang, dass ein durchgehender Schallwellenzug sich zwischen den beiden, in der Messzelle vorgesehenen Schallempfängern befindet. Dabei sind die hier eingesetzten Schallempfänger ausreichend phasenempfindlich, also zeitauflösend, dass der Phasenversatz bei bekanntem Abstand der beiden Schallempfänger und bekannter Schallfrequenz zur Bestimmung der Schallgeschwindigkeit eingesetzt werden kann.

Für Überprüfungs- oder Korrelationszwecke ist es natürlich möglich, noch eine größere Anzahl von Schallempfängern in der Messzelle anzuordnen, die Länge der kontinuierlich abgestrahlten Schallwelle (kleinste Wellenlänge) ist dabei so bestimmt, dass die doppelte Distanz zwischen zwei in der Messzelle vorgesehenen Schallempfängern nicht unterschritten und auch nicht überschritten wird. Dabei sind die einsetzbaren Schallempfänger ausreichend phasenempfindlich, das heißt, zeitauflösend, dass ein Phasenversatz bei bekanntem Abstand zwischen den Schallempfängern und bekannter Schallfrequenz zur Bestimmung der Schallgeschwindigkeit verwendet werden kann. Durch eine konstante Anregungsfrequenz hat das Signal auch an zwei Schallempfängern die selbe sinusförmige Frequenz. In der Auswerteeinheit werden beiden Signale bevorzugt einem Phase-Lock-In-Verstärker zugeführt und für jedes der Signale ein Rechtecksignal abgeleitet. Somit haben die Rechtecksignale die selbe Phasenverschiebung, wie die Sinussignale und können zeitlich leicht ausgewertet werden. Um Reflektionen zu vermeiden, die zu einer undefinierten zusätzlichen Phasenverschiebung führen würden, ist es als günstig gefunden worden, wenn am Ende der Messzelle ein langer Schlauch mit dem selben Innendurchmesser, wie der Innendurchmesser der Messzelle, angebracht ist. In diesem Schlauch, der auch als Rohr oder dergleichen ausgebildet sein kann, läuft sich die Schallwelle quasi reflektionsfrei tot. Der Schlauch kann dabei auf einem Wickel angeordnet werden, sodass das Gerät insgesamt kompakt aufgebaut werden kann.

Diese Variante erlaubt es auch, dass die Schallquelle in den Abstrahlpausen als Schallempfänger dient. Die Länge der Schallwellenanregung beziehungsweise des Schallwellenimpulses beträgt dabei bevorzugt 2 bis 5 Perioden auf der Resonanzfrequenz des Schallgebers, sodass nach der Anregung die Schallquelle abgeschaltet wird und nun als Schallempfänger für das Empfangssignal dient. Die zu erwartende Schalllaufzeit wird dabei günstigerweise so eingestellt, dass zumindest kurz vor Eintreffen des Empfangssignals der Schallgeber ausgeschwungen und empfindlich genug für die Registrierung des Empfangssignales ist.

In dem hier geschilderten Fall wird der Zeitpunkt der Emission der Schallquelle wie auch der Aufnahme des Empfangssignales durch das gleiche Gerät als Zeitpunkt der Auswerteeinheit zur Bestimmung der Schallgeschwindigkeit zugeleitet. Bei der verhältnismäßig einfach ausgestalteten Messzelle, die die Erfindung umfasst, wird dabei dieses Schallwellenimpulspaket von einer Begrenzungsfläche der Messzelle reflektiert. Gegebenenfalls ist in der Messzelle auch eine Schallwellen gut absorbierende Oberfläche vorgesehen, um zusätzliche Reflektionen zu vermeiden.

In einer Variante ist/sind in der Messzelle eine oder mehrere Reflektionsflächen für die Schallwellen vorgesehen. Deren Abstand beziehungsweise Position untereinander beziehungsweise zueinander sind fertigungstechnisch gut beherrschbar und daher mit hoher Güte kostengünstig herstellbar. Gerade die Kombination einer Mehrzahl von Reflektionsflächen mit dem Konzept der Emission von Schallwellenimpulsen führt zu einer Vielzahl von zeitlich versetzt am Schallempfänger auftretenden Empfangssignalen, die durch Einsatz von Kreuzkorrelationsrechnungen zur Bestimmung der Schallgeschwindigkeit mit hoher Qualität und Güte führen.

Eine weitere Variante der Messzelle, die als vorteilhaft angesehen wird, erlaubt eine zusätzliche Messung des Druckes und/oder der Temperatur des Gases und sieht die Berücksichtigung dieser Messwerte beziehungsweise deren Auswirkung auf die Schallgeschwindigkeit im Auswerteprozess vor. Damit ist es möglich, die Konzentration der entsprechenden Gase in einem laufenden Prozess unter Druck zu messen. Vorteilhaft an dieser Variante der Messzelle ist, dass schwankende Drücke in der Messzelle sich nicht auf die Konzentrationsbestimmung auswirken.

Die auch als Puls-Echo-Methode bekannte Variante erlaubt es im Auswerteprozess auch die Lage der Schallquelle zu den Reflektionsflächen zu berücksichtigen. Es resultiert hieraus eine Unabhängigkeit der Schallquelle von der Reflektionsfläche. Im Auswerteprozess wird die Laufzeit bezüglich der Lage der Schallquelle zu den Reflektionsflächen und die Differenzen der Reflektionsflächen untereinander, die in der Regel bekannt sind, bestimmt und es resultiert daraus die Unabhängigkeit der Lage der Schallquelle zu den Differenzabständen der Reflektionsflächen. Ein entsprechender Einstellaufwand oder Kalibriervorgang für die Positionierung der Schallquelle (beziehungsweise des Schallempfängers) wird damit vermieden beziehungsweise ist nur bei einem bekannten Gasgemisch, einer bekannten Temperatur und einem bekannten Druck erforderlich, was die Herstellungskosten senkt beziehungsweise günstig beeinflusst.

Der Ansatz setzt Schallwellen ein, also phonetische Wellen, die sich in dem Gasgemisch beziehungsweise Gas ausbreiten. Bevorzugterweise wird dabei Schall einer Frequenz beziehungsweise einer Wellenlänge (monochromatisch) eingesetzt, um den Messaufwand gering zu halten. Grundsätzlich ist es möglich, die erfindungsgemäße Anordnung mit Ultraschall zu betreiben, wird aber in der ersten Variante vorgeschlagen, die Schallwellen im hörbaren Frequenzbereich anzuregen.

Insbesondere ist die Frequenz der Schallwellen kleiner 10 kHz, bevorzugt kleiner 3 kHz. Gute Ergebnisse wurden insbesondere bei einer Frequenz von ca. 1,5 kHz (+/- 15%) erreicht.

Eine weitere, als vorteilhaft angesehene Weiterbildung der Erfindung sieht, wie oben ausgeführt, vor, dass Schallwellen im nicht hörbaren Frequenzbereich angeregt werden. Insbesondere ist die Frequenz der Schallwellen dann kleiner als 500 kHz, bevorzugt kleiner als 300 kHz.

Es wurde bereits darauf hingewiesen, dass es in einer Variante günstig ist, dass in der Messzelle ein Temperatursensor und/oder ein Drucksensor vorgesehen ist. Diese sind über eine Temperatur- und/oder Druckmessleitung mit der Auswerteinheit datentechnisch verbunden und übergeben über diese Messdaten an die Auswerteinheit.

Der Einsatz eines Temperatur- oder Drucksensors dient nicht nur dazu, den Betriebsbereich der Vorrichtung zu überwachen, sondern erlaubt es auch, die bestehende Temperatur- oder Druckabhängigkeit der Schallgeschwindigkeit zu berücksichtigen. Das Messergebnis wird damit genauer und fehlerfreier.

Des Weiteren umfasst die Erfindung nicht nur die Vorrichtung wie beschrieben, sondern auch ein Verfahren zur Bestimmung des Anteiles von SF6- und/oder CF4-Gas in einer Gasmischung, wobei zunächst eine Messzelle mit dem Gasgemisch gefüllt wird, dann eine an der Messzelle angeordnete Schallquelle eine permanente Schallwelle in das Gasgemisch der Messzelle abstrahlt und mindestens zwei an der Messzelle angeordneter phasenempfindliche Schallempfänger die Schallwelle phasenaufgelöst aufnehmen und das jeweilige Empfangssignal einer Auswerteinheit zugeleitet wird und die Auswerteinheit zunächst die Schallgeschwindigkeit aus dem Phasenunterschied der Empfangsignale ermittelt und hieraus den Anteil von SF6- und/oder CF4-Gas im Gasgemisch bestimmt.

In einer bevorzugten Ausgestaltung des Verfahrens registriert die Auswerteinheit den Abstrahlzeitpunkt des Abstrahlens der Schallwelle. Auch erhält die Auswerteinheit den Zeitpunkt des Eintreffens des Empfangssignals und kann durch einen Vergleich des Abstrahlzeitpunktes mit dem Zeitpunkt des Empfangssignals die Schallgeschwindigkeit (bei bekannter Laufstrecke) ermitteln. Dabei ist es möglich, dass der Abstrahlzeitpunkt beziehungsweise das Starten des Schallwellenimpulses von der Auswerteinheit vorgegeben wird, also die Auswerteinheit auch Steuerungsaufgaben übernimmt oder aber mit Beginn des Abstrahlens der Schallwelle (Schallwellenimpulses) von der Schallquelle auch ein entsprechendes Signal an die Auswerteinheit übergeben wird.

Des Weiteren umfasst das Verfahren in einer Variante auch die Möglichkeit, die Schallgeschwindigkeit durch (Auto-)Kreuzkorrelation der von mehreren Reflektionsflächen in der Messzelle verursachten Empfangssignale zu bestimmen. Durch eine solche Verfahrensweise wird zum Einen die Genauigkeit gesteigert und die Ergebnisse überprüft (alle Einzelergebnisse der an den Differenzabständen der Reflektionsflächen berechneten Schallgeschwindigkeiten müssen exakt den selben Wert liefern) und zum Anderen werden gleichzeitig fertigungsbedingte Ungenauigkeiten eliminiert, wodurch der Herstellungsaufwand sinkt.

Eine Weiterbildung des Verfahrens sieht vor, dass in der Auswerteeinheit eine dritte oder weitere Reflektionsfläche(n) vorgesehen ist/sind und mit deren Hilfe eine Überprüfung des Ergebnisses der Schallgeschwindigkeitsmessung durchgeführt wird. Diese Überprüfung erfolgt in definierten zeitlichen Abständen beziehungsweise in einem definierten zeitlichen Abstand, beispielsweise nach jeder Messung oder nach einer Serie von Messungen. Die Überprüfung der Messung der Schallgeschwindigkeit erfolgt dabei aus einer Laufzeitdifferenzmessung für die jeweiligen Reflektionsflächen.

Eine weitere Verbesserung des Messverfahrens stellt es dar, wenn die Energie, das heißt, die Amplitude der in das Gasgemisch der Messzelle abgestrahlten Schallwelle durch entsprechende Ansteuerung des Schallgebers an die Dichte des Gasgemisches angepasst wird. Diese Anpassung kann dabei variabel erfolgen und auch das Ergebnis einer insbesondere vorab durchgeführten Test- oder Kalibriermessung darstellen.

In einer Weiterbildung des Verfahrens ist es vorgesehen, dass die Konzentration, das heißt, die Konzentration von SF6- und/oder CF4-Gas in einem Gasgemisch im laufenden Prozess atmosphärisch, das heißt, unter atmosphärischen Druckbedingungen oder unter Druck durchgeführt wird.

Die Anteile von SF6- und/oder CF4-Gas in einem Gasgemisch können dabei auch, wie in einer weiteren günstigen Variante des Verfahrens vorgesehen, direkt aus der Schallgeschwindigkeit oder indirekt über die Laufzeiten der Schallwellen oder die Laufzeitdifferenzen bestimmt werden. Hierdurch stellt das erfindungsgemäße Verfahren mehrere Bestimmungsarten für die Anteile/Konzentrationen von Gasen beispielsweise in einem Gasgemisch zur Verfügung, die alternativ oder parallel in einer Messzelle verwirklicht werden können.

In der Zeichnung ist die Erfindung insbesondere in einem Ausführungsbeispiel schematisch dargestellt. Es zeigen:

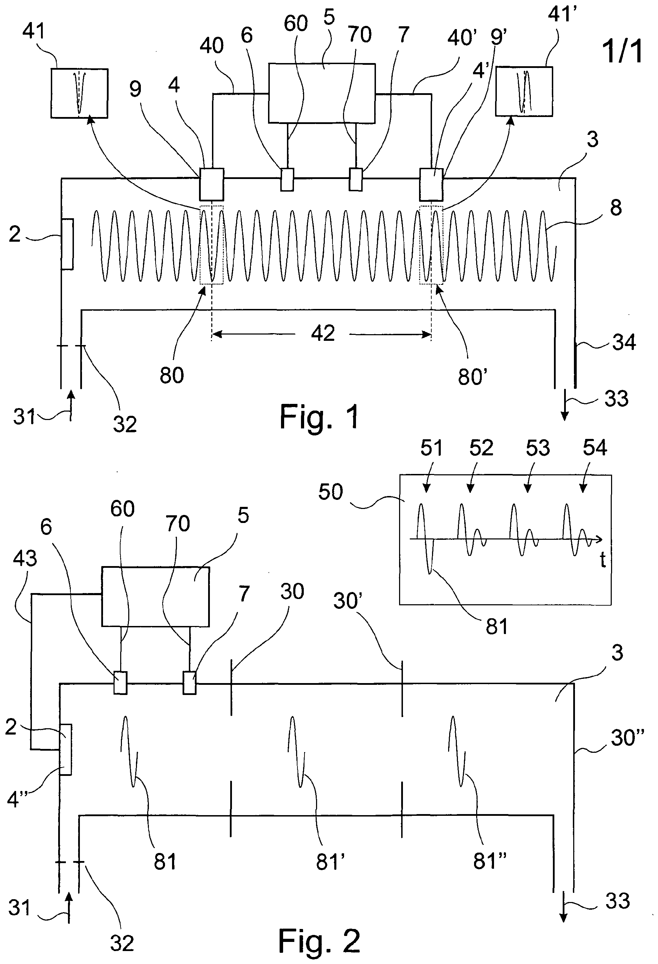

Fig. 1, 2 je eine schematische Darstellung der Vorrichtung.
In den Figuren sind gleiche oder einander entsprechende Elemente jeweils mit den gleichen Bezugszeichen bezeichnet und werden daher, sofern nicht zweckmäßig, nicht erneut beschrieben.

In Fig. 1 ist eine Variante der Vorrichtung gezeigt. Die gezeigte Vorrichtung dient zur Bestimmung des Anteiles von SF6- und/oder CF4-Gas in einem Gasgemisch, wobei sich das Gasgemisch in einer Messzelle 3 befindet und an der Messzelle 3 eine Schallquelle 2 und zwei Schallempfänger 4, 4' angeordnet sind. Die Messzelle 3 weist hierzu Bohrungen 9, 9' für die Ankopplung der Schallempfänger 4, 4' auf. Die beiden Schallempfänger 4, 4' liegen eine bekannte Distanz 42 voneinander entfernt. Die Distanz ist abhängig von der Anregungsfrequenz und ist so gewählt, dass sich zwischen beiden Schallempfängern 4, 4' bei der kleinsten Schallgeschwindigkeit (größte SF6-Gas-Konzentration) und der tiefsten möglichen Messgastemperatur eine Phasenverschiebung von etwa Ò, der sich ausbreitenden Schallwelle zwischen den Schallempfängern 4, 4' ergibt. Phasenverschiebungen größer Ò sind mehrdeutig und unbrauchbar. Dabei nehmen die beiden Schallempfänger 4, 4' den Momentanzustand der von der Schallquelle 2 abgestrahlten Schallwelle 8 auf und leiten das Empfangssignal über die Datenleitungen 40, 40' an eine Ansteuer- und Auswerteeinheit 5, die letztlich die Konzentration in dem sich in der Messzelle 3 befindlichen Gasgemisch aus der Phasenverschiebung und damit der Schallgeschwindigkeit bestimmt. Hieraus ermittelt dann die Ansteuer- und Auswerteeinheit 5 auch den Anteil von SF6- und/oder CF4-Gas in dem Gasgemisch.

Dies bedeutet beispielsweise, dass zur Messung einer SF6-Konzentration von 100 Vol-% bei einer Anregungsfrequenz von 1,536 kHz ein Abstand von 41,5 mm zwischen den Schallempfängern 4, 4' ideal ist, um den möglichen Messbereich voll ausnutzen zu können.

Die Ausgestaltung der Messzelle 3 ist dabei so gewählt, dass diese im Durchflusskonzept betreibbar ist. Hierzu weist die Messzelle 3 einen Gaszulauf 31 und einen Gasablauf 33 auf. Gegebenenfalls sind der Gaszulauf 31 und/oder der Gasablauf 33 durch separate, hier nicht dargestellte, Absperrhähne absperrbar. Im Gaszulauf 31, noch vor der Messzelle 3, ist eine Einlassblende 32 als den Druck drosselndes Element vorgesehen. Am Gasausgang 33 dient ein dort angeordneter Wickelschlauch 34 zur Vermeidung von Reflexionen.

In dem in Fig. 1 aber auch in Fig. 2 gezeigten Ausführungsbeispiel ist die Messzelle 3 verhältnismäßig lang erstreckend realisiert, wobei die Schallquelle 2 an einer Stirnseite der Messzelle angeordnet ist. In dem in Fig. 1 gezeigten Ausführungsbeispiel wird eine Permanentschallwelle 8 von der Schallquelle 2 abgestrahlt.

Die beiden Schallempfänger 4, 4' sind ausreichend empfindlich, um die Phasenlage der jeweiligen Schallwellenanteile 80, 80' zeitlich aufzulösen. Es ergibt sich dann das in den kleinen Zusatzbildern dargestellte Empfangssignal 41, 41'. Mit der gestrichelten Linie ist in den Fenstern der Empfangssignale 41, 41' der gleichzeitige Messzeitpunkt angedeutet. Es ist die unterschiedliche Phasenlage bei dem im ersten Schallempfänger 4 und im zweiten Schallempfänger 4' aufgenommenen Empfangssignal 41, 41' zu erkennen. Aufgrund dieser Information und der (monochromatischen) Schallfrequenz der Schallwelle 8 sowie dem bekannten Abstand 42 der beiden Schallempfänger 4, 4' ist es möglich, in der Ansteuer- und Auswerteinheit 5 die Schallgeschwindigkeit und hieraus auch die Gaszusammensetzung des in der Messzelle 3 vorgehaltenen Gasgemisches zu bestimmen. Die Auswerteeinheit 5 ist beispielsweise als Mikrokontroller und/oder DSP realisiert und dient als Ansteuer- und Auswerteeinheit 5.

Der direkte, numerische Messwert der Schallgeschwindigkeit ist dabei nicht von Bedeutung, vielmehr wir durch Kalibrierung der Vorrichtung mit Prüfgasen unterschiedlicher Gaskonzentration über den gesamten Betriebstemperaturbereich die Phasenverschiebung bei einzelnen, konstanten Temperaturen aufgetragen. Durch diese einzelnen Punkte werden kubisches Splinepolynome berechnet, um auch Phasenverschiebungen in Zwischenbereichen numerisch bestimmen zu können. Mit Hilfe der als Mikrokontroller und/oder DSP ausgebildeten Ansteuer- und Auswerteeinheit 5 beziehungsweise des in der Ansteuer- und Auswerteeinheit 5 vorgesehenen Mikrokontrollers und/oder DSP, wird durch Messung der Phasenverschiebung und der Temperatur durch lineare Interpolation der Messwerte die Gaskonzentration in der Messzelle 3 bestimmt. In verschiedenen Varianten der Erfindung kann der Messwert digital zur Anzeige gebracht werden und/oder aber über ein Bussystem weiteren Teilnehmern und/oder Ansteuer- oder Auswerteinheiten 5 zur Verfügung gestellt werden.

Für Überwachungszwecke, aber auch um das Messergebnis entsprechend anzupassen, sind an der Messzelle 3 auch ein Temperatursensor 6 sowie ein Drucksensor 7 optional angeordnet. Die Schallgeschwindigkeit ist unter Umständen abhängig von dem Temperatur- und Druckniveau des Gasgemisches, weswegen es günstig ist, eine entsprechende Druck- und/oder Temperaturüberwachung einzurichten. Hierzu ist die Ansteuer- und Auswerteinheit 5 mit dem Temperatursensor 6 über die Temperaturmessleitung 60 und der Drucksensor 7 über die Druckmessleitung 70 verbunden.

Die Lage des Temperatursensors 6 beziehungsweise des Drucksensors 7 in der Messzelle 3 ist variabel. Dieser kann zum Beispiel mittig oder auch randseitig angeordnet sein.

In Fig. 2 ist ein alternatives, nicht erfindungsgemäßes Konzept der Vorrichtung gezeigt. Hier wird eine zylindrische Messzelle 3 verwendet, deren grundsätzlicher Aufbau dem nach Fig. 1 entspricht. Die Messzelle 3 ist jedoch in keinster Weise auf die zylindrische Form beschränkt.

Die Gesamtlänge der Messzelle 3 nach de r Erfindung beträgt beispielsweise zwischen 5 cm und 15 cm.

Im Gegensatz zu dem Konzept nach Fig. 1 ist bei Fig. 2 die Schallquelle 2 auch gleichzeitig Schallempfänger 4''. Dies ist möglich, da das Konzept zum Erzeugen einer Schallwelle mit der Schallquelle 2 umgekehrt betrieben zu einem Schallempfänger wird, und so in einem Gerät zwei Funktionen kostengünstig realisierbar sind. Dabei ist zu beachten, dass in dem in Fig. 2 gezeigten Ausführungsbeispiel die Schallquelle 2 keine permanente Schallwelle 8 erzeugt, sondern nur Schallwellenimpulse 81, 81', 81''. Die Betriebsweise der Vorrichtung 2 ist dabei als Puls-Echo-Methode beschreibbar. Dabei wird von der Schallquelle 2 ein Schallwellenimpuls 81, 81', 81'' in das Gasgemisch der Messzelle 3 abgestrahlt. Unter Schallewellenimpuls ist ein Impulspaket von 2 bis 5 Perioden auf der Resonanzfrequenz der Schallquelle 2 beziehungsweise des Schallgebers zu verstehen, auch wenn in Fig. 2 symbolisch nur eine Periode dargestellt ist. Die Schallquelle 2 beziehungsweise der Schallgeber sendet periodisch in gewissen zeitlichen Abständen einen Schallwellenimpuls aus und geht nach Abklingen der Schwingung auf Empfang. Während der Abstrahlpause zwischen zwei hintereinanderliegenden Schallwellenimpulsen 81, 81', 81'' dient die Schallquelle 2 daher als Schallempfänger 4''.

In der Messzelle 3 sind mehrere Reflektionsflächen 30, 30', 30'' vorgesehen, an welchen der Schallwellenimpuls 81, 81', 81'' reflektiert wird. Wenn nun drei Reflektionsflächen 30, 30', 30'' vorgesehen sind, so gelangen zeitlich versetzt drei Antwortsignale zu dem Schallempfänger 4''. Der Abstand der Reflektionsflächen 30, 30', 30'' untereinander wie auch der Abstand zur Schallquelle 2/Schallempfänger 4'' ist bekannt. Auch ist der Zeitpunkt des Abstrahlens des Schallwellenimpulses 81 in der Ansteuer- und Auswerteinheit 5 hinterlegbar (dies erfolgt über die Ansteuer- und Informationsleitung 43), über die auch die Reflektionssignale, die von dem Schallempfänger 4'' aufgenommen werden, an die Auswerteinheit 5 gelangen. Der typische Signalverlauf nach der Informationsleitung 43 ist in dem Fenster 50 angedeutet. 51 kennzeichnet dabei den von der Schallquelle 2 abgestrahlten Schallwellenimpuls 81. 52 kennzeichnet das reflektierte Signal der ersten Reflektionsfläche 30, das Reflektionssignal der zweiten Reflektionsfläche 30' ist mit 53 und das Reflektionssignal der dritten Reflektionsfläche 30'' mit 54 gekennzeichnet. Der zeitliche Abstand der einzelnen Reflektionssignale wird gemessen.

Die Reflektionsflächen 30, 30' sind dabei als scheiben- oder blendenartige Elemente in der Messzelle 3 angeordnet, als Reflektionsfläche 30'' kann aber auch die Abschlusswand der Messzelle 3 selber dienen. Der Abstand untereinander, aber auch der Abstand zur Schallquelle 2/Schallempfänger 4'' ist bekannt. Somit ist die Schallgeschwindigkeit durch die Ansteuer- und Auswerteinheit 5 ermittelbar, und hierüber, aus einer entsprechenden Vergleichstabelle oder angepassten Formel auch der Gasanteil in dem Gasgemisch.

## Patentansprüche

1. Vorrichtung zur Bestimmung des Anteils von SF6-und/oder CF4-Gas in einem Gasgemisch, wobei sich das Gasgemisch in einer Messzelle befindet und an der Messzelle eine zum Abstrahlen einer kontinuierlichen Schallwelle eingerichtete Schallquelle und mindestens ein zur Aufnahme der Schallwelle eingerichteter Schallempfänger angeordnet sind und wobei die Vorrichtung eine Auswerteeinheit umfasst, die dafür eingerichtet ist dass ihr das Empfangssignal des mindestens einen Schallempfängers zugeleitet wird, und dass sie die Schallgeschwindigkeit und daraus resultierend den Anteil von SF6-beziehungsweise CF4-Gas in dem Gasgemisch bestimmt, **dadurch gekennzeichnet, dass** mindestens zwei, untereinander und auch von der Schallquelle (2) entfernte, phasenempfindliche Schallempfänger (4, 4') an der Messzelle (3) vorgesehen sind und dafür eingerichtet sind, die Schallwellen (8) phasenaufgelöst zu empfangen und das jeweilige Empfangssignal (41, 41') der Auswerteeinheit (5) zuzuleiten und die Auswerteeinheit (5) dafür eingerichtet ist aus dem Phasenunterschied der jeweiligen Empfangssignale (41, 41') die Schallgeschwindigkeit und hieraus den Anteil des jeweiligen Gases zu bestimmen, wobei die Länge der kontinuierlich abgestrahlten Schallwelle mindestens so lang ist, dass ein durchgehender Schallwellenzug sich zwischen den beiden in der Messzelle vorgesehenen Schallempfängern befindet.

2. Vorrichtung nach Anspruch 1, **gekennzeichnet durch** einen Gaszu-(31) und einen Gasablauf (33) der Messzelle (3), wobei bevorzugt im Gaszulauf (31) eine Einlassblende (32) vorgesehen ist und wobei insbesondere in der Einlassblende (32) wenigstens eine Bohrung, bevorzugt im µm-Bereich, vorgesehen ist und/oder durch eine Vorrichtung zum Auffangen des Messgases, insbesondere im Gasablauf (33) .

3. Vorrichtung nach einem oder beiden der vorhergehenden Ansprüche, **dadurch gekennzeichnet, dass** die Messzelle (3) für einen Messdruck von 1,0 bis 1,7 bar, insbesondere 1,2 bis 1,7 bar, bevorzugt atmosphärischen Druck, ausgelegt ist.

4. Vorrichtung nach einem oder mehreren der vorhergehenden Ansprüche, **gekennzeichnet durch** eine Gasrückführung am Gasablauf (33) und/oder eine maximale Durchsatzmenge durch die Messzelle (3) bei 100% SF6 von weniger als 2,4 g/Min. bevorzugt weniger als 1,8 g/Min., insbesondere weniger als 1,5 g/Min. und/oder einen Betriebsdruck in der Zuleitung zum Gaszulauf (31) von bis zu 20 bar, insbesondere von bis zu 15 bar, bevorzugt von bis zu 12 bar und/oder, insbesondere vor der Einlassblende (32) von bis zu 10 bar.

5. Vorrichtung nach einem oder mehreren der vorhergehenden Ansprüche, **dadurch gekennzeichnet, dass** sie für eine Messung bei strömenden Gas ausgelegt ist, wobei vorzugsweise eine Fliessgeschwindigkeit des Gasgemisches durch die Messzelle (3) vorgesehen ist, die deutlich geringer ist als die Schallgeschwindigkeit im Gasgemisch.

6. Vorrichtung nach einem oder mehreren der vorhergehenden Ansprüche, **gekennzeichnet durch** eine länglich ausgebildete Messzelle (3) mit einer daraus resultierenden verhältnismässig langen Messstrecke, wobei bevorzugt die Schallquelle (2), insbesondere in Abstrahlpausen, auch als Schallempfänger (4") dienen kann.

7. Vorrichtung nach einem oder mehreren der vorhergehenden Ansprüche, **dadurch gekennzeichnet dass** die Schallquelle auch geeignet ist Schallwellenimpulse (81) und/oder eine Frequenz der Schallwellen (8) im hörbaren Frequenzbereich, insbesondere kleiner 10 kHz, bevorzugt kleiner 3 kHz abzustrahlen.

8. Vorrichtung nach einem oder mehreren der vorhergehenden Ansprüche, **gekennzeichnet durch** eine Frequenz der Schallwellen (81) im nicht hörbaren Frequenzbereich, insbesondere kleiner als 500 kHz, bevorzugt kleiner als 300 kHz.

9. Vorrichtung nach einem oder mehreren der vorhergehenden Ansprüche, **dadurch gekennzeichnet, dass** in der Messzelle (3) ein Temperatursensor (6) vorgesehen ist, der über eine Temperaturmessleitung (60) für die Übergabe von Messdaten mit der Auswerteeinheit (5) datentechnisch verbunden ist und/oder in der Messzelle (3) ein Drucksensor (7) vorgesehen ist, der über eine Druckmessleitung (70) für die Übergabe von Messdaten mit der Auswerteeinheit (5) datentechnisch verbunden ist und/oder in der Messzelle (3) eine oder mehrere Reflexionsflächen (30, 30', 30") für die Schallwelle/n (81) vorgesehen ist/sind.

10. Vorrichtung nach einem oder mehreren der vorhergehenden Ansprüche, **dadurch gekennzeichnet, dass** die Schallquelle (2) auch dafür eingerichtet ist einen Schallwellenimpuls; (81) in das Gasgemisch in der Messzelle (3) abzustrahlen und während der Abstrahlpause als Schallempfänger (4'') zu dienen und die Auswerteeinheit (5) dafür eingerichtet ist den Abstrahlzeitpunkt festzuhalten und in der Messzelle (3) eine oder mehrere Reflexionsflächen (30, 30', 30") vorgesehen sind sodass der Schallempfänger (4") die an der/den Reflexionsfläche/n (30, 30', 30") reflektierte(n) Schallwellenimpuls/e (81) empfängt und das/die Empfangssignal/e der Auswerteeinheit (5) zuleitet, welche dafür eingerichtet ist aus der/den Laufzeit/en die Schallgeschwindigkeit, und hieraus den Anteil des jeweiligen Gases zu bestimmen.

11. Stromleittechnische, gastechnische oder schalltechnische Anlage mit einer Vorrichtung nach einem oder mehreren der vorhergehenden Ansprüche.

12. Verfahren zur Bestimmung des Anteils von SF6- und/ oder CF4-Gas in einem Gasgemisch mittels einer Vorrichtung gemäß einem der Ansprüche 1 bis 11, wobei zunächst die Messzelle mit dem Gasgemisch gefüllt wird oder das Gasgemisch kontinuierlich die Messzelle durchströmt, dann die, an der Messzelle angeordnete Schallquelle eine kontinuierliche Schallwelle in das Gasgemisch der Messzelle abstrahlt, und mindestens zwei, an der Messzelle angeordneter phasenempfindliche Schallempfänger die Schallwelle phasenaufgelöst aufnehmen und das jeweilige Empfangssignal einer Auswerteeinheit zugeleiten und die Auswerteeinheit zunächst die Schallgeschwindigkeit aus dem Phasenunterschied der Empfangsignale ermittelt und hieraus, insbesondere über Tabellenwerte oder Formeln, den Anteil von SF6- und/oder CF4-Gas im Gasgemisch bestimmt, wobei die Länge der kontinuierlich abgestrahlten Schallwelle mindestens so lang ist, dass ein durchgehender Schallwellenzug sich zwischen den beiden in der Messzelle vorgesehenen Schallempfängern befindet.

13. Verfahren nach Anspruch 12, **dadurch gekennzeichnet, dass** die Auswerteeinheit den Abstrahlzeitpunkt des Abstrahlens der Schallwellen registriert und mit dem Zeitpunkt des Empfangssignales die Schallgeschwindigkeit ermittelt und/oder die Schallgeschwindigkeit durch (Auto-)Kreuz-korrelation der von den wenigstens zwei Reflektionsflächen in der Messzelle verursachten Empfangssignale bestimmt und/oder die Energie der durch die Schallquelle (2) abgegebenen Schallwelle, insbesondere die Amplitude der Schallwelle, bevorzugt durch Testmessung, an die Dichte des Gasgemisches variabel angepasst wird.

14. Verfahren nach Anspruch 13, **dadurch gekennzeichnet, dass** eine dritte oder weitere Reflektionsfläche(n) (30, 30', 30") vorgesehen ist/sind und die Auswerteeinheit (5) mit Hilfe der dritten oder weiteren Reflektionsfläche(n) (30, 30', 30") in definiertem und/oder definierten zeitlichen Abstand/Abständen das Ergebnis der Messung der Schallgeschwindigkeit durch wenigstens eine Laufzeitdifferenzmessung überprüft wird.

15. Verfahren nach einem oder mehreren der vorhergehenden Ansprüche 12 bis 14, **dadurch gekennzeichnet, dass** der Anteil von SF6- und/oder CF4-Gas in einem Gasgemisch in einem laufenden Prozess atmosphärisch oder unter Druck gemessen wird und/oder die Anteile von SF6- und/oder CF4-Gas in dem Gasgemisch aus der Schallgeschwindigkeit direkt oder indirekt über die Laufzeiten der Schallwellen und/oder über die Laufzeitdifferenzen der Schallwellen bestimmt werden.

## Claims

1. Device for measuring the amount of SF6 and/or CF4 gas in a gas mixture, wherein the gas mixture is in a measuring cell, and at the measuring cell a sound source, adapted for emitting a continuous sound wave, and at least one sound receiver, adapted for receiving the sound wave, are arranged, and wherein the device comprises an evaluating unit, adapted such that the receive signal of the at least one sound receiver is guided thereto, and that it determines the sound velocity and resulting thereof the amount of SF6 or CF4 gas in the gas mixture, **characterized in that** at least two phase-sensitive sound receivers (4, 4'), that are distanced from one another and also from the sound source (2), are provided at the measuring cell (3) and adapted for receiving the sound waves (8) phase-resolvedly and for guiding the respective receive signal (41, 41') to the evaluating unit (5), and the evaluating unit (5) is adapted for determining the sound velocity und thereof the amount of the respective gas by means of the phase difference of the respective receive signals (41, 41'), wherein the continuously emitted sound wave has at least such a length that a continuous sound wave train is between the two sound receivers provided in the measuring cell.

2. Device according to claim 1, **characterized by** a gas feed (31) and a gas outlet (33) of the measuring cell (3), wherein preferably in the gas feed (31) a feed screen (32) is provided, and wherein in particular in the feed screen (32) at least one bore hole, preferably in the µm range, is provided, and/or by a device for collecting the measuring gas, in particular in the gas outlet (33).

3. Device according to one or both of the preceding claims, **characterized in that** that the measuring cell (3) is dimensioned for a measuring pressure of 1.0 to 1.7 bar, in particular 1.2 to 1.7, preferably atmospheric pressure.

4. Device according to one or more of the preceding claims, **characterized by** a gas recirculation at the gas outlet (33) and/or a maximum throughput through the measuring cell (3) at 100% SF6 of less than 2.4 g/min, preferably less than 1.8 g/min, in particular less than 1.5 g/min, and/or an operational pressure in the supply of the gas feed (31) of up to 20 bar, in particular of up to 15 bar, preferably of up to 12 bar, and/or in particular upstream the feed screen (32) of up to 10 bar.

5. Device according to one or more of the preceding claims, **characterized in that** it is dimensioned for measuring while the gas flows, wherein preferably a flow velocity of the gas mixture through the measuring cell (3) is provided that is clearly less than the sound velocity in the gas mixture.

6. Device according to one or more of the preceding claims, **characterized by** a longitudinally formed measuring cell (3) with a resulting rather long measuring section, wherein preferably the sound source (2), in particular during breaks of emission, may serve as sound receiver (4'').

7. Device according to one or more of the preceding claims, **characterized in that** the sound source is also suitable for emitting bursts of sound waves (81) and/or a frequency of the sound waves (8) in the audible frequency range, in particular less than 10 kHz, preferably less than 3 kHz.

8. Device according to one or more of the preceding claims, **characterized by** a frequency of the sound waves (81) in the non-audible frequency range, in particular less than 500 kHz, preferably less than 300 kHz.

9. Device according to one or more of the preceding claims, **characterized in that** the measuring cell (3) a temperature sensor (6) is provided that is data-connected via a temperature measuring line (60) to the evaluating unit (5) for transmitting measuring data, and/or in the measuring cell (3) a pressure sensor (7) is provided that is data-connected via a pressure measuring line (70) to the evaluating unit (5) for transmitting measuring data, and/or in the measuring cell (3) one or more reflection surfaces (30, 30', 30'') is/are provided for the sound waves(s) (81).

10. Device according to one or more of the preceding claims, **characterized in that** the sound source (2) is also adapted for emitting a burst of sound wave (81) into the gas mixture in the measuring cell (3) and serving as sound receiver (4'') during the emitting break, and the evaluating unit (5) is adapted for holding the emitting moment, and in the measuring cell (3) one or more reflection surfaces (30, 30', 30'') is/are provided so that the sound receiver (4'') receives the burst(s) of the sound waves (81) reflected at the reflection surface(s) (30, 30', 30''), and transmits the receive signal(s) to the evaluating unit (5) that is adapted for determining the sound velocity from the travel time(s), and thereof the amount of the respective gas.

11. Apparatus for the implementation of current conduction, gas or sound with a device according to one or more of the preceding claims.

12. Method for measuring the amount of SF6 and/or CF4 gas in a gas mixture by means of a device according to one of the claims 1 to 11, wherein first the measuring cell is filled with the gas mixture or the gas mixture continuously flows through the measuring cell, then the sound source, arranged at the measuring cell, emits a continuous sound wave into the gas mixture of the measuring cell, and at least two phase-sensitive sound receivers, arranged at the measuring cell, receive the sound wave phase-resolvedly and transmit the respective receive signal to an evaluating unit, and first the evaluating unit determines the sound velocity from the phase difference of the receive signals and thereof defines, in particular through table values or formulas, the amount of SF6 and/or CF4 gas in the gas mixture, wherein the length of the continuously emitted sound wave has at least such a length that a continuous sound wave train is between the two sound receivers provided in the measuring cell.

13. Method according to claim 12, **characterized in that** the evaluating unit records the moment of emission of the sound waves and determines along with the moment of the receive signal the sound velocity, and/or determines the sound velocity by means of (auto) cross-correlation of the receive signals caused by the at least two reflection surfaces in the measuring cell, and/or the energy of the sound wave emitted by the sound source (2), in particular the amplitude of the sound wave, is variably adapted, preferably by test measurement, to the density of the gas mixture.

14. Method according to claim 13, **characterized in that** a third or other reflection surface(s) (30, 30', 30'') is/are provided, and the evaluating unit (5) verifies by means of the third or other reflection surface(s) (30, 30', 30'') in defined and/or temporally defined interval(s) the result of the measurement of the sound velocity by at least one measurement of the travel time difference.

15. Method according to one or more of the preceding claims 12 to 14, **characterized in that** the amount of SF6 and/or CF4 gas in a gas mixture during an ongoing process is measured atmospherically or under pressure, and/or the amount of SF6 and/or CF4 gas in the gas mixture is determined from the sound velocity directly or indirectly through the travel time of the sound waves and/or the travel time differences of the sound waves.

## Revendications

1. Dispositif destiné à la détermination d'une teneur en gaz SF6 et/ou CF4 dans un mélange de gaz situé à l'intérieur d'une cellule de mesure comportant une source d'ondes acoustiques permettant d'envoyer une onde acoustique en continu et au moins un récepteur acoustique façonné pour recevoir cette onde acoustique avec une unité d'analyse située au niveau du dispositif configurée de façon à recevoir le signal de réception d'au moins un récepteur acoustique lui permettant de déterminer la vitesse de l'onde acoustique et, résultant de cette vitesse, la teneur en gaz de SF6 ou de CF4 dans le mélange de gaz, **caractérisé en ce qu'**au moins deux récepteurs acoustiques (4, 4') sensibles à la phase du signal acoustique, séparés entre eux et par rapport à la source acoustique (2), sont prévus au niveau de la cellule de mesure (3) et configurés de façon à recevoir les ondes acoustiques (8) et leurs phases leur permettant de transmettre chacun ces signaux reçus (41, 41') à l'unité d'analyse (5) laquelle peut analyser la différence de phases entre les signaux reçus (41, 41') et déterminer la vitesse acoustique et ainsi la teneur de chacun des gaz et ceci, avec une longueur d'onde acoustique émise en continu au moins équivalente à la longueur entre les deux récepteurs acoustiques prévus à l'intérieur de la cellule de mesure.

2. Dispositif selon la revendication 1, **caractérisé par** une entrée de gaz (31) et par une sortie de gaz (33) de la cellule de mesure (3) et de préférence par la présence d'un diaphragme (32) au niveau de l'entrée de gaz (31) avec en particulier une ouverture de préférence de taille micrométrique et/ou par un dispositif d'accumulation du gaz mesurée en particulier au niveau de la sortie de gaz (33).

3. Dispositif selon une ou selon les deux revendications précédentes, **caractérisé en ce que** la cellule de mesure (3) est conçue pour une pression atmosphérique pendant la mesure allant de 1,0 à 1,7 bar et de préférence de 1,2 à 1,7 bar.

4. Dispositif selon une ou plusieurs des revendications précédentes, **caractérisé par** l'existence d'un retour de gaz au niveau de la sortie de gaz (33) et/ou par un débit maximal de la cellule de mesure (3) lors de la présence de 100% de gaz SF6 de moins de 1,4g/min et de préférence moins de 1,8 g/min et en particulier moins de 1,5g/min et/ou par une pression opérationnelle dans l'arrivée de l'entrée de gaz (31) allant jusqu'à 20 bar, en particulier jusqu'à 15 bar et de préférence jusqu'à 12 bar et/ou en particulier devant le diaphragme (32) jusqu'à 10 bar.

5. Dispositif selon une ou plusieurs revendications précédentes, **caractérisé en ce qu'**il est configuré pour la mesure d'un flux de gaz.

6. Dispositif selon une ou plusieurs des revendications précédentes, **caractérisé par** une cellule de mesure en longueur (3) augmentant ainsi la distance de mesure et avec une configuration préférée permettant d'utiliser la source acoustique (2) surtout lors des pauses d'émission également comme récepteur acoustique (4'').

7. Dispositif selon une ou plusieurs des revendications précédentes, **caractérisé en ce que** la source acoustique est également capable d'envoyer des impulsions d'onde acoustique (81) et/ou une fréquence d'onde acoustique (8) dans la gamme de fréquences audibles et en particulier inférieure à 10 kHz et de préférence inférieure à 3kHz.

8. Dispositif selon une ou plusieurs des revendications précédentes, **caractérisé par** une fréquence des ondes acoustiques (81) située en dehors des fréquences audibles, en particulier inférieure à 500kHz et de préférence inférieure à 300kHz.

9. Dispositif selon une ou plusieurs revendications précédentes, **caractérisé en ce qu'**un capteur de température (6) est prévu à l'intérieur de la cellule de mesure (3) lié à l'unité d'analyse (5) via une ligne de mesure de température (60) afin de transmette les données de température et/ou **en ce qu'**un capteur de pression (7) est prévu à l'intérieur de la cellule de mesure (3) lié à l'unité d'analyse (5) via une ligne de mesure de pression (70) afin de transmette les données de pression et/ou **en ce qu'**une ou plusieurs surfaces de réflexion (30, 30', 30'') pour la ou les onde (s) acoustique (s) (81) est(sont) prévue(s).

10. Dispositif selon une ou plusieurs des revendications précédentes, **caractérisé en ce que** la source acoustique (2) est également configurée afin de pouvoir envoyer une impulsion d'onde acoustique (81) dans le mélange de gaz dans la cellule de mesure (3) et de servir lors de la pause d'émission comme récepteur acoustique (4'') et **en ce que** l'unité d'analyse (5) est configurée pour saisir le moment d'émission et **en ce qu'**une ou plusieurs surfaces de réflexion (30, 30', 30'') est(sont) prévue(s) à l'intérieur de la cellule de mesure (3) afin que le récepteur acoustique (4'') puisse recevoir la(les) impulsion(s) acoustique(s) (81) réfléchie(s) à la(les) surface(s) réfléchissante(s) (30, 30', 30'') et **en ce que** le(les) signal(signaux) de réceptionest(sont) transmis à l'unité d'analyse (5) qui est configurée afin de pouvoir déterminer la vitesse acoustique à partir du(des) temps de parcours et ainsi la teneur de chacun des gaz.

11. Installation technique électrique, à gaz ou acoustique comportant un dispositif selon une ou plusieurs des revendications précédentes.

12. Procédé pour la détermination de la teneur en gaz de SF6 et/ou de CF4 dans un mélange de gaz à l'aide d'un dispositif selon une des revendications 1 à 11 en remplissant d'abord la cellule de mesure avec le mélange de gaz ou en faisant traverser la cellule de mesure par le mélange de gaz en continu puis en envoyant une onde acoustique continuepar la source acoustique située à l'intérieur de la cellule de mesure à travers le mélange de gaz vers au moins deux récepteurssensibles à la phase du signal acoustique qui déterminent les phases du signal puis envoient les signaux reçus vers une unité d'analyse qui détermine d'abord la vitesse acoustique par la différence des phases des signaux reçus puis traduit en particulier à l'aide de tableaux de correspondance ou de formules la teneur de SF6 et/ou de CF4 gaz dans le mélange de gazet ceci dans une configuration permettant de placer au moins une longueur d'onde acoustique continuecomplète entre les deux récepteurs prévus à l'intérieur de la cellule de mesure.

13. Procédé selon la revendication 12, **caractérisé en ce que** l'unité d'analyse enregistre le moment d'envoi des ondes acoustiques et détermine avec celui-ci et avec le moment de réception la vitesse acoustique et/ou **en ce que** la vitesse acoustique est déterminée par corrélation croisée (automatique) des signaux reçus réfléchis par au moins deux surfaces de réflexion à l'intérieur de la cellule de mesure et/ou **en ce que** l'énergie de l'onde acoustique envoyée par la source acoustique (2) et en particulier l'amplitude de l'onde acoustique est adaptée de façon variable à la densité du mélange de gaz de préférence à l'aide de mesures de test.

14. Procédé selon la revendication 13, **caractérisé en ce qu'**une troisième ou d'autres surfaces de réflexion (30, 30' 30'') sont prévues et **en ce que** l'unité d'analyse (5) contrôle le résultat des mesures de la vitesse acoustique par au moins une mesure de temps de parcours différentielle à un et/ou des moment(s) défini(s) à l'aide de la troisième ou d'autres surfaces de réflexion (30, 30', 30'').

15. Procédé selon une ou plusieurs des revendications précédentes 12 à 14, **caractérisé en ce que** la teneur de gaz SF6 et/ou de gaz CF4 dans le mélange de gaz est mesurée selon un procédé continu à pression atmosphérique ou sous pression et/ou **en ce que** la teneur de SF6 et/ou de CF4 dans le mélange de gaz est déterminée à partir de la vitesse acoustique directement ou indirectement à l'aide des temps de parcours des ondes acoustiques et/ou à l'aide des différences de temps de parcours des ondes acoustiques.
